# EUROPEAN PATENT APPLICATION

(11) **EP 4 512 437 A1**
(43) Date of publication of application: **26.02.2025**
(21) Application number: 23792015.2
(22) Date of filing: 03.03.2023
(51) Int. Cl.: A61L 27/36

(54) **MULTI-STAGE DECELLULARIZED TISSUE SUPPLEMENT, AND PREPARATION METHOD THEREOF**

(30) Priority: 21.04.2022 KR 20220049749
(71) Applicant: T&R Biofab Co., Ltd., Siheung-si Gyeonggi-do 15111 (KR)
(72) Inventor: KIM, Hyun Jung, Uiwang-si, Gyeonggi-do 16024 (KR); KIM, Chang Hwan, Siheung-si, Gyeonggi-do 14985 (KR); PARK, Eun Hye, Ansan-si, Gyeonggi-do 15521 (KR); KIM, Young Hwan, Suwon-si, Gyeonggi-do 16313 (KR); SHIN, Jae Hang, Siheung-si, Gyeonggi-do 15032 (KR); CHOI, In Hank, Hwaseong-si, Gyeonggi-do 18585 (KR)
(74) Representative: Caspary, Karsten
(86) International application number: PCT/KR2023/002940
(87) International publication number: WO 2023/204434

(57) **Abstract**

The present invention relates to a multi-stage decellularized mammal-derived tissue supplement, prepared by a method comprising: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid, and wherein the DNA content in the tissue subjected to the pretreatment step to the second inactivation step is 50 ng/mg or less, and the degradation time using 100 U/ml of collagenase is at least 90 hours; and a method for preparing the same. The tissue supplement of the present invention has the advantage that DNA and crude fat are almost removed, and the degradation time and tensile stress can be controlled, and therefore it can be customized to the site to be transplanted.

## Description

### [Technical Field]

The present invention relates to a multi-stage decellularized tissue supplement and a method for preparing the same, and more specifically, to a multi-stage decellularized tissue supplement having low DNA content and crude fat content and capable of controlling mechanical properties and degradation time by multi-stage decellularization, and a method for preparing the same.

### [Background Art]

In general, biotransplantable tissue supplements are used to replace, treat or restore human tissues or organs that have been lost due to accidents or diseases. These tissue supplements mimic the structure and mechanical behavior of the tissues or organs to be transplanted to restore the biomechanical function of the damaged tissues, so that they should have a certain strength, support cell migration, adhesion, proliferation and differentiation, be biocompatible so as not to induce an immune response, have low antigenicity, and degrade at a rate similar to or equal to the rate of new tissue formation.

Biotransplantable tissue supplements are classified into synthetic tissue supplements and biological tissue supplements, and these tissue supplements can be used as artificial tissues or organs for research, or as organ models for new drug development, in addition to biotransplantation.

Synthetic tissue supplements are obtained by synthesizing polymers, and are prepared to not elicit an immune response and to mimic the structure of tissue supplements using biocompatible polymers such as biocompatible fibrous polymers, gelatin, collagen, poly latic acid (PLA) or poly glycolic acid (PGA), but when tissue supplements having a three-dimensional structure with a predetermined thickness are prepared and used, there is a problem that oxygen and nutrients cannot be sufficiently supplied to all cells, and there is a problem that it is not easy to maintain and manage the cells within the tissue supplements, and therefore, synthetic tissue supplements have the problem that they are limited in thickness or size and are not sufficient to perform functions equivalent to or similar to living tissues or organs.

Due to the limitations of these synthetic tissue supplements, tissue supplements derived from animal tissues have recently been in the spotlight. Tissue supplements derived from animal tissues have the advantage of superior biological function because they can be applied in a pre-formed size, thereby having excellent biomechanical function, and provide an environment very suitable for cell behavior after transplantation.

Tissue supplements derived from animal tissues contain substances that induce immune or inflammatory responses, such as cells, fats, viruses, and other foreign substances, so that the most important part in preparing tissue supplements derived from animal tissues is the process of removing these foreign substances. To remove these substances, acid, alkali or enzyme treatments are generally used, but no method has yet been developed that can efficiently remove all cells, fats, viruses and other foreign substances.

In addition, there are disadvantages in that the yield of tissue supplements from which substances causing immune or inflammatory responses have been removed is low and the processing time is long.

Meanwhile, the required strength and degradation period vary depending on the type of tissue or organ to which the biotransplantable tissue supplements are applied, but tissue supplements derived from animal tissues prepared by conventional methods have the problem that it is difficult to control the strength and degradation period.

### [Disclosure]

### [Technical Problem]

The present invention aims to provide a multi-stage decellularized tissue supplement having low DNA content and crude fat content and capable of controlling mechanical properties and degradation time by multi-stage decellularization, and a method for preparing the same.

### [Technical Solution]

One embodiment of the present invention for achieving the above-described purposes relates to a decellularized tissue supplement, and the tissue supplement is prepared by a method comprising: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, the DNA content in the tissue subjected to the pretreatment step to the second inactivation step may be 50 ng/mg or less, and the degradation time using 100 U/ml of collagenase may be at least 90 hours, preferably 90 to 750 hours.

The primary decellularization step may comprise a first decellularization step using a mixture of a aqueous basic solution and an alcohol; and a second decellularization step using a aqueous basic solution.

The tissue may be any one or more of mammal-derived skin, cartilage, ligament, tendon, myocardial membrane, pericardium, small intestine, bladder, liver, heart, lung, pancreas, spleen, kidney, stomach, bronchus, uterus, placenta and nerve.

The first decellularization step may be performed using a mixture of n-PrOH and NaOH, and the second decellularization step may be performed using NaOH of 0.05 M or more and less than 0.5 M.

The secondary decellularization step may be performed using DNase of less than 0.03 wt%.

Another embodiment of the present invention relates to a method for preparing a decellularized tissue supplement, and specifically, comprises: a preparation step for preparing skin tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, the DNA content in the tissue subjected to the pretreatment step to the second inactivation step may be 50 ng/mg or less, and the degradation time using 100 U/ml of collagenase may be at least 90 hours, preferably 90 to 750 hours.

The primary decellularization step may comprise a first decellularization step using a mixture of a aqueous basic solution and an alcohol; and a second decellularization step using a aqueous basic solution.

A neutralization step using an acidic solution and a washing step using PBS may be further performed between the primary and secondary decellularization steps.

A decolorization step and/or a defatization step may be further performed between the secondary decellularization step and the second inactivation step.

The second inactivation step may be performed using a mixture of an organic acid and an alcohol.

Washing, processing, packaging and sterilization steps may be performed after the second inactivation step.

The pretreatment step to the second inactivation step may be performed in a stirring environment of less than 150 rpm.

### [Advantageous Effects]

The multi-stage decellularized tissue supplement of the present invention may be customized to the human organ into which the tissue supplement is to be transplanted because the DNA content and crude fat content are low and the degradation time and physical strength may be controlled.

In addition, the method for preparing a multi-stage decellularized tissue supplement has the advantage of being able to process about twice as much at one time as the conventional method because the decellularization efficiency is improved through the multi-stage decellularization process, and significantly improving productivity because the processing time is short.

In addition, it has the advantage of being able to remove most of the DNA in a short period of time even when a lower concentration of DNA-degrading enzyme is used because the decellularization efficiency is improved.

### [Description of Drawings]

Figure 1 shows the results of analyzing H&E staining photographs taken according to Experimental Example 1.
Figure 2 shows the results of analyzing H&E staining photographs taken according to Experimental Example 2.
Figure 3 is a graph showing the tensile stress with respect to strain in Experimental Example 3.
Figure 4 shows the results of analyzing H&E staining photographs taken according to Experimental Example 4.

### [Best Mode]

Before explaining in detail the preferred embodiments of the present invention below, it should be noted that the terms and words used in this specification and claims should not be interpreted as limited to their usual or dictionary meanings, but should be interpreted as meanings and concepts consistent with the technical spirit of the present invention.

Throughout this specification, when a part is said to "comprise" or "include" an element, it means that other elements may be further included without excluding other elements, unless otherwise stated.

Throughout this specification, "%" used to indicate the concentration of a particular substance means (weight/weight) % for solid/solid, (weight/volume) % for solid/liquid, and (volume/volume)% for liquid/liquid, unless otherwise stated.

In addition, throughout this specification, the term "mammal" refers to a non-human mammal, and should be understood to mean a non-human mammal even if the "mammal" is simply described without mentioning that a human is excluded, and the expression "cells are removed" should be understood to have an inclusive meaning including that DNA is destroyed or removed.

In addition, each of the steps may be performed in a different order than specified, unless a specific order is clearly described from the context. That is, each of the steps may be performed in the same order as specified, may be performed substantially simultaneously, or may be performed in the opposite order.

In addition, unless otherwise defined, all technical and scientific terms used in this specification have the same meaning as commonly understood by those of ordinary skill in the technical field to which the present invention pertains, and in case of conflict, the description in this specification, including definitions, shall prevail.

Hereinafter, embodiments of the present invention will be described. However, the scope of the present invention is not limited to the preferred embodiments below, and those skilled in the art can implement various modified forms of the contents described in this specification within the scope of the present invention.

The present invention relates to a multi-stage decellularized mammal-derived tissue supplement and a method for preparing the same, and the multi-stage decellularized mammal-derived tissue supplement of the present invention is prepared by removing cells, a-gal epitopes, viruses, crude fats, and other foreign substances of mammal-derived connective tissue through a multi-stage decellularization process, and the tissue supplement thus prepared has an advantage in that it may be customized to breast reconstruction, burns, rotator cuff tears, hernias, plastic surgery, etc., because the degradation time in the body may be controlled.

First, the multi-stage decellularized mammal-derived tissue supplement according to one embodiment of the present invention is prepared by a method comprising: a preparation step for preparing skin tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, it is characterized in that the DNA content in the tissue subjected to the pretreatment step to the second inactivation step may be 50 ng/mg or less, and the degradation time using 100 U/ml of collagenase may be at least 90 hours, preferably 90 to 750 hours.

As such, the multi-stage decellularized mammal-derived tissue supplement of the present invention has a low DNA content, so that it hardly induces an immune response or foreign body reaction, and the degradation time using collagenase can be adjusted from a short time to a long time as described above, so that a tissue supplement of the present invention having a different degradation time may be customized to the human body site to which the tissue supplement is applied.

First, the preparation step is a step of preparing tissue of a non-human mammal. In this case, the mammal may be a non-human mammal, for example, a pig, a horse, a cow, a sheep, etc., and the tissue may be any one or more of mammal-derived skin, cartilage, ligament, tendon, myocardial membrane, pericardium, small intestine, bladder, liver, heart, lung, pancreas, spleen, kidney, stomach, bronchus, uterus, placenta and nerve.

To prepare a multi-stage decellularized tissue supplement, mammal tissue is first collected. The mammal tissue thus collected is treated by removing useless tissue and blood attached to the mammal tissue, washing, drying and cutting in the preparation step. The mammal tissue thus prepared may be stored frozen and may be thawed and used when work is required. When frozen raw material tissue is used, the preparation step may be a step of taking out and preparing the frozen raw material tissue.

Next, the mammal tissue prepared in the preparation step is pretreated through the pretreatment step.

This step is a step of washing the mammal tissue, and if the mammal tissue is frozen, it may be thawed, washed and cut through this step. In this step, distilled water, purified water, saline solution, etc. may be used as the washing water, and preferably, distilled water is used.

In addition, physical stirring may be performed from this step to the second inactivation step, wherein the physical stirring is preferably performed at a stirring speed of less than 150 rpm. This is because if the physical stirring speed is 150 rpm or more, the tissue may be damaged and may become difficult to use.

The mammal tissue prepared through the pretreatment step may be subjected to the first inactivation step, primary decellularization step, secondary decellularization step and second inactivation step, thereby removing cells, crude fats, viruses and other foreign substances that may induce immune responses and foreign body reactions. By decellularization through such a multi-stage reaction, the decellularization efficiency and foreign substance removal efficiency at each step are remarkably improved, so that the amount of tissue processed at one time increases and the processing time is shortened, and as a result, the productivity and yield of the decellularized tissue supplement may be improved. In addition, by changing some of the multi-stage reaction conditions, the degradation time may be controlled from tens to hundreds of hours.

The first inactivation step is performed after the pretreatment step, and an additional step may be further performed between the two steps to remove the epidermis or basement membrane. This step may be performed in such a way as to enzymatically treat or physically separate the tissue.

The first inactivation step is a step of inactivating a virus included in the pretreated tissue using alcohol.

This step is performed to more effectively remove cells, fats and foreign substances in the primary and secondary decellularization steps by primarily inactivating a virus within the tissue prior to decellularization of the tissue.

The alcohol used in this step may be n-propanol, and an aqueous n-propanol solution with a concentration of 50 to 90% is preferably used to obtain a virus inactivation effect, and an aqueous n-propanol solution with a concentration of 65 to 80% may be more preferably used.

Ethanol is generally used for virus inactivation, but in the present invention, n-propanol is used as the alcohol for virus inactivation in the first inactivation step. When n-propanol is used for virus inactivation in this way, the tissue becomes softer, and thus, it has strength, elasticity and texture similar to connective tissue, like actual human skin.

In addition, when the tissue is soft, the treatment solution penetrates better into the tissue, and thus, an effect is obtained that allows for overall uniform performance of virus inactivation and decellularization. As a result, the efficiency of inactivation and decellularization treatment is improved, more uniform tissue may be obtained, and the quality and reliability of the finally obtained decellularized tissue supplement may be improved.

Ethanol has the characteristic of fixing and hardening tissue, but if ethanol is used in this step, there is a problem that since the tissue becomes hard, the difference in shape and texture from human connective tissue increases, and there is a problem that since the tissue becomes loose in the subsequent step, the efficiency of the process of improving the penetration ability of the treatment material decreases. In addition, iso-propanol has a low virus inactivation effect, and if iso-propanol is used in the first inactivation step, since sufficient virus inactivation effect is not obtained, the decellularization efficiency is reduced in the subsequent primary and secondary decellularization steps, and therefore, it is preferable to use n-propanol.

In this step, the pretreated tissue may be treated in an amount of 10 to 25 parts by weight, preferably 20 to 25 parts by weight, based on 100 parts by weight of the aqueous propanol solution.

The primary decellularization step is a step of treating the virus-inactivated tissue from the first inactivation step with an aqueous basic solution to remove cells.

This step comprises a first decellularization step for first treating the tissue using a mixture of an aqueous basic solution and alcohol; and a second decellularization step for treating the tissue using an aqueous basic solution. In this case, the aqueous basic solution is preferably an aqueous sodium hydroxide solution, and the alcohol is preferably n-propanol, and in this case, the first decellularization step is the step performed by treating the tissue with a mixture of n-propanol and sodium hydroxide, and the second decellularization step is the step performed by treating the tissue with an aqueous sodium hydroxide solution.

When the decellularization step is performed in two steps like this, since not only is decellularization performed, but also foreign substances such as crude fat are removed through the first decellularization step, a more effective decellularization process is performed in the second decellularization step.

In addition, in this step, the physical strength, such as tensile stress and suture strength of the tissue, and the degradation rate may be controlled depending on the processing conditions. In particular, the present invention does not use a separate cross-linking agent throughout the entire process, and since the tensile stress or suture strength may be controlled by changing some of the processing conditions without using a cross-linking agent in this way, there is an advantage in that a tissue supplement with a desired strength and degradation rate may be prepared in a simpler and more efficient manner.

In each of the first and second decellularization steps, the tissue may be treated in an amount of 10 to 25 parts by weight, preferably 20 to 25 parts by weight, based on 100 parts by weight of the treatment solution, which is nearly twice the conventional treatment capacity, and since effective decellularization may be performed through the multi-stage method of the present invention, there is an effect that allows for sufficient treatment of twice the amount of tissue compared to the conventional method.

Specifically, the first decellularization step is a step of treating the virus-inactivated tissue with a mixture of n-propanol and sodium hydroxide. In this step, the crude fat and sodium hydroxide in the tissue undergo a saponification reaction, and the crude fat is separated and removed from the tissue, wherein n-propanol promotes this saponification reaction and acts to cause the products of the saponification reaction to coagulate with each other, allowing the crude fat to be separated and removed from the tissue more quickly and efficiently.

In this case, the treatment time for treating the tissue with a mixture of n-propanol and sodium hydroxide may be 10 to 48 hours, preferably 15 to 40 hours, and more preferably 18 to 28 hours, and the treatment temperature may be 0 to 30°C, preferably 10 to 28°C, and more preferably 18 to 25°C, and when treated within this temperature range for this time, sufficient fat removal efficiency may be obtained without significant damage to the tissue structure.

The mixture of n-propanol and sodium hydroxide may be may be an aqueous solution with a concentration of n-propanol of 50 to 95%, preferably 60 to 90%, more preferably 65 to 85%, and a concentration of sodium hydroxide of 0.05 M or more and less than 0.5 M, and these concentration ranges are concentration ranges for minimizing tissue damage and removing foreign substances such as crude fat in the first decellularization step, and in particular, if the concentration of sodium hydroxide is less than this range, the treatment efficiency of the primary decellularization step is reduced, and if it is outside this range, there is a problem that the tissue structure itself collapses and dissolves, and therefore, it is preferable to use an n-propanol and aqueous sodium hydroxide solution in the concentration ranges described above.

The second decellularization step is a step for simultaneously causing a decellularization reaction while loosening the structure of the tissue by treating the tissue subjected to the first decellularization step with an aqueous basic solution to soften the tissue. Crude fats and various foreign substances included in the tissue act as a kind of physical and chemical barrier in the decellularization reaction, and since the tissue was subjected to the first decellularization step and the crude fats and various foreign substances were removed, the decellularization reaction may be performed more effectively in the second decellularization step.

In this step, the treatment time for treating the tissue with an aqueous basic solution may be 10 to 48 hours, preferably 15 to 40 hours, and more preferably 18 to 28 hours, and the treatment temperature may be 0 to 30°C, preferably 10 to 28°C, and more preferably 18 to 25°C, and when treated within this temperature range for this time, the tissue structure may be sufficiently loosely deformed without collapse of the tissue structure or loss of elastin, and an appropriate decellularization reaction may occur.

In this case, the aqueous basic solution used as the treatment solution may be an aqueous sodium hydroxide solution, and the concentration of sodium hydroxide included in the aqueous sodium hydroxide solution may be 0.05 M or more and less than 0.5 M. If the concentration of sodium hydroxide is less than 0.05 M, since the tissue structure is not sufficiently loosened, there is a problem that the decellularization efficiency decreases in the secondary decellularization step described later, and if it is 0.5 M or more, since some tissues may collapse or dissolve beyond the level of loosening in this step, the final yield may decrease remarkably, and therefore, it is preferable to use an aqueous sodium hydroxide solution of the concentration described above.

The secondary decellularization step using enzymes is performed after the primary decellularization step, and a neutralization step and a washing step may be performed between these two steps.

The neutralization step is a step of treating the tissue with an aqueous acidic solution to neutralize the sodium hydroxide used in the primary decellularization step, and an aqueous acidic solution containing any one or more of, for example, but not limited to, hydrochloric acid, sulfuric acid, acetic acid and peracetic acid may be used as the type of aqueous acidic solution used, and the concentration thereof may also be appropriately adjusted and used according to the working environment or conditions.

The washing step is the step performed to prevent a decrease in enzyme reactivity due to residues when enzymatically treated in the subsequent secondary decellularization step, and a buffer solution may be used as the washing solution used in the washing step, and for example, a phosphate buffered saline (PBS) solution may be used, but is not limited.

Meanwhile, the secondary decellularization step is a step of enzymatically treating the primary decellularized tissue to remove cells, and the step of treating the primary decellularized tissue with a DNA-degrading enzyme to remove DNA within the primary decellularized tissue.

In this step, the tissue may be treated in an amount of 10 to 25 parts by weight, preferably 20 to 25 parts by weight, based on 100 parts by weight of the treatment solution containing the DNA-degrading enzyme, which is nearly twice the conventional treatment capacity as described above and is an achievable treatment capacity because the treatment efficiency is increased by the multi-stage decellularization treatment process of the present invention.

In this step, the treatment time for treating the tissue with the DNA-degrading enzyme to obtain enzyme activity and sufficient DNA degradation efficiency may be 10 to 35 hours, preferably 18 to 30 hours, and the treatment temperature may be 30 to 45°C, preferably 35 to 42°C.

In the present invention, the concentration of the DNA-degrading enzyme contained in the treatment solution may be less than 0.03 wt%, which is at least several times lower than the concentration of the DNA-degrading enzyme used conventionally. The primary decellularized tissue of the present invention is subjected to the primary decellularization step, wherein foreign substances including crude fats within the tissue are removed and the structure of the tissue becomes loose. In this state, when a DNA-degrading enzyme is added, the DNA-degrading enzyme may penetrate very easily into the tissue structure, and accordingly, even if a low concentration of the DNA-degrading enzyme is used, at least the same or better DNA removal efficiency may be secured, and therefore, a low concentration of the DNA-degrading enzyme as described above may be used in the present invention.

However, if the concentration of the DNA-degrading enzyme is 0.03 wt% or more, since the degree of improvement in DNA degradation efficiency is extremely small compared to the amount of the added DNA-degrading enzyme, it is uneconomical, and therefore, it is preferable to use the DNA-degrading enzyme in the concentration range described above.

The decellularized tissue from the secondary decellularization step is subjected to the second inactivation step to remove an additional virus, and additional decolorization and/or defatization steps may be performed between these two steps. The decolorization step is a step of removing the color of the tissue, and may be performed, for example, by treating the tissue with hydrogen peroxide, and the defatization step is a step of further removing fat included in the tissue, and may be performed, for example, using a ketone solution, preferably an acetone solution.

Subsequently, the second inactivation step is performed to further inactivate a virus included within the tissue subjected to the secondary decellularization step. This step is a step of inactivating a virus within the tissue using an acid, specifically the step of treating the tissue with a mixture of an organic acid and an alcohol.

In this case, the organic acid used may be peracetic acid, wherein the peracetic acid is an oxidizing agent and may inactivate a virus such a way as to cause non-specific free radical damage to the virus. The peracetic acid used in this step may be used at a concentration of 0.05 to 1%.

In addition, the alcohol used in this step may be a lower alcohol having 1 to 4 carbon atoms, such as methanol, ethanol and propanol, and preferably, may be ethanol, and since ethanol has the function of inactivating a virus and the function of fixing a tissue structure to make the tissue firm, it is preferable to use ethanol.

Through these steps, the virus is finally inactivated, the crude fat and DNA are removed, and a tissue supplement comprising various extracellular matrix components may be obtained.

The tissue supplement thus obtained has a very low DNA content of 50 ng/mg or less, so that the induction of immune responses or various foreign body reactions may be minimized.

Meanwhile, additional steps such as washing, processing, packaging and sterilization may be performed after the second inactivation step. The washing may be performed using a buffer solution, distilled water, etc., and the processing may be a process of cutting it into a form that is easy to use, transport or package, or a process of perforating it to form holes or slits.

The packaging is performed to prevent contamination and facilitate handling during storage or transport of the tissue supplement, and may be packaged with a preservative, if necessary.

The sterilization is performed to remove any additional contamination that may occur after the second inactivation step, and for example, but not limited to, electron beam irradiation or radiation irradiation may be performed for sterilization.

Meanwhile, another embodiment of the present invention relates to a method for preparing a multi-stage decellularized mammal-derived tissue supplement, and some of the description overlapping with one embodiment of the present invention is omitted.

The method for preparing a decellularized tissue supplement comprises: a preparation step for preparing skin tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, it is characterized in that the DNA content in the tissue subjected to the pretreatment step to the second inactivation step may be 50 ng/mg or less, and the degradation time using 100 U/ml of collagenase may be at least 90 hours, preferably 90 to 750 hours.

First, the preparation step is a step of preparing tissue of a non-human mammal. In this case, the mammal may be a non-human mammal, for example, a pig, a horse, a cow, a sheep, etc., and the tissue may be any one or more of skin, cartilage, ligament, tendon, myocardial membrane, pericardium, small intestine, bladder, liver, heart, lung, pancreas, spleen, kidney, stomach, bronchus, uterus, placenta and nerve derived from such mammals.

The pretreatment step is a step of washing the mammal tissue prepared in the preparation step, and the tissue may be cut into an appropriate size at this step, if necessary, and when previously frozen tissue is prepared, thawing may be performed at this step. In addition, physical stirring may be performed from this step to the second inactivation step, wherein the physical stirring is preferably performed at a stirring speed of less than 150 rpm.

The first inactivation step is performed after the pretreatment step, and an additional step may be further performed between the two steps to remove the epidermis or basement membrane. This step may be performed in such a way as to enzymatically treat or physically separate the tissue.

The first inactivation step is a step of inactivating a virus included in the pretreated tissue using alcohol, and ethanol is generally used for virus inactivation, n-propanol is used in the present invention. This is because ethanol firmly fixes the tissue to reduce the softening efficiency in the subsequent step, whereas n-propanol softens the tissue to improve the softening efficiency in the subsequent step, while giving it a texture, properties, strength, elasticity, etc. more similar to connective tissue such as human skin.

The primary decellularization step is a step of treating the virus-inactivated tissue from the first inactivation step with an aqueous basic solution to remove cells, and through this step, not only are cells removed, but the tissue becomes soft, so that the texture, properties, strength, elasticity, etc. of the tissue change to become more similar to connective tissue such as human skin. In particular, this effect is achieved by using an aqueous basic solution of a specific concentration, and the degradation time of the tissue supplement may be controlled from tens to hundreds of minutes by changing the concentration of the aqueous basic solution during the primary decellularization step.

This step comprises a first decellularization step for first treating the tissue using a mixture of an aqueous basic solution and alcohol; and a second decellularization step for treating the tissue using an aqueous basic solution.

In this case, it is preferable to use an aqueous sodium hydroxide solution as the aqueous basic solution and n-propanol as the alcohol.

Specifically, the first decellularization step is a step of treating the virus-inactivated tissue with a mixture of n-propanol and sodium hydroxide, and through this step, crude fats and foreign substances within the tissue are removed and the tissue is partially softened. The second decellularization step is a step of treating the tissue with an aqueous sodium hydroxide solution, and in this step, a decellularization reaction occurs simultaneously with tissue softening, and this effect may be further enhanced by removing foreign substances including crude fat in advance.

In the first and second decellularization steps, sodium hydroxide may be included in a concentration of 0.05 M or more and less than 0.5 M, which is a desirable concentration range for preventing tissue collapse or dissolution and forming strength, elasticity, etc. similar to human skin while sufficiently causing saponification and softening reactions by sodium hydroxide.

Next, the secondary decellularization step using enzymes is performed after the primary decellularization step, and a neutralization step and a washing step may be performed between these two steps. The neutralization step is a step of treating the tissue with an aqueous acidic solution to neutralize the sodium hydroxide used in the primary decellularization step, and the washing step is a step performed to prevent a decrease in enzyme reactivity due to residues when enzymatically treated in the subsequent secondary decellularization step.

The secondary decellularization step is a step of treating the primary decellularized tissue with a DNA-degrading enzyme to remove DNA within the tissue. The primary decellularized tissue of the present invention is subjected to the primary decellularization step, wherein foreign substances including crude fats within the tissue are removed and the structure of the tissue becomes loose. In this state, when a DNA-degrading enzyme is added, the DNA-degrading enzyme may penetrate very easily into the tissue structure, and accordingly, even if a low concentration of the DNA-degrading enzyme is used, at least the same or better DNA removal efficiency may be secured, and therefore, the DNA-degrading enzyme is used at a low concentration of less than 0.03 wt% in the present invention. If the concentration of the DNA-degrading enzyme is greater than or equal to this rage, since the degree of improvement in DNA degradation efficiency is extremely small compared to the amount of the added DNA-degrading enzyme, it is uneconomical, and therefore, it is preferable to use the DNA-degrading enzyme in the concentration range described above.

The decellularized tissue from the secondary decellularization step is subjected to the second inactivation step to remove an additional virus, and additional decolorization and/or defatization steps may be performed between these two steps. The decolorization step is a step of removing the color of the tissue, and may be performed, for example, by treating the tissue with hydrogen peroxide, and the defatization step is a step of further removing fat included in the tissue, and may be performed, for example, using a ketone solution, preferably an acetone solution.

Subsequently, the second inactivation step is performed to further inactivate a virus included within the tissue subjected to the secondary decellularization step. This step is a step of treating the tissue with a mixture of an organic acid and an alcohol, wherein a peracetic acid may be used as the organic acid and ethanol may be used as the alcohol, and a virus within the tissue may be effectively removed by treating the tissue with this mixed solution.

Through these steps, the virus is finally inactivated, the crude fat and DNA are removed, and a tissue supplement comprising various extracellular matrix components may be prepared.

The tissue supplement thus obtained has a very low DNA content of 50 ng/mg or less, so that when the tissue supplement is transplanted into the human body, the induction of immune responses or various foreign body reactions may be minimized.

Meanwhile, additional steps such as washing, processing, packaging and sterilization may be performed after the second inactivation step. The washing may be performed using a buffer solution, distilled water, etc., and the processing may be a process of cutting it into a form that is easy to use, transport or package, or a process of perforating it to form holes or slits.

The packaging is performed to prevent contamination and facilitate handling during storage or transport of the tissue supplement, and may be packaged with a preservative, if necessary.

The sterilization is performed to remove any additional contamination that may occur after the second inactivation step, and for example, but not limited to, electron beam irradiation or radiation irradiation may be performed for sterilization.

Hereinafter, the specific action and effect of the present invention will be explained through an example of the present invention. However, this is presented as a preferred example of the present invention, and the scope of the present invention is not limited according to the example.

### [Preparative Examples]

### 1. Preparation and pretreatment steps

First, washed, dried, cut and frozen pig skin was prepared, thawed, washed with distilled water, and immersed in an enzyme treatment solution containing 0.1 wt% lipase and 1 M NaCl at 25°C for 2 hours to prepare a raw material tissue specimen (pig dermis) with the epidermis and basement membrane removed.

### 2. First inactivation step

Next, 230 g of the raw material tissue specimen was added to 1 L of 70% n-propanol and stirred at a temperature of 20°C and a stirring speed of 50 RPM for 24 hours to perform the first inactivation step.

### 3-1. Primary decellularization step (first decellularization step)

Next, a mixed solution containing 70% concentration of n-propanol and 0.1 M concentration of sodium hydroxide in distilled water was prepared, and the raw material tissue specimen was mixed to contain 22 parts by weight based on 100 parts by weight of the mixed solution, and then stirred at a temperature of 20°C and a stirring speed of 50 RPM for 24 hours to perform the first decellularization step.

### 3-2. Primary decellularization step (second decellularization step)

Next, the tissue specimen subjected to the first decellularization step was mixed to contain 24 parts by weight based on 100 parts by weight of a pre-prepared 0.1 M aqueous sodium hydroxide solution, and then stirred for 24 hours under the same conditions as the previous step to perform the second decellularization step.

Subsequently, the tissue specimen subjected to the second decellularization step was immersed in an aqueous acetic acid solution, stirred to neutralize, and then washed with a PBS solution.

### 4. Secondary decellularization step

Subsequently, the tissue specimen was placed in a treatment solution containing DNase at a concentration of 0.003 wt% and stirred at a temperature of 37°C and a stirring speed of 50 RPM for 22 hours to perform a secondary decellularization step. The tissue specimen subjected to the secondary decellularization step was decolorized by treating it in 3% hydrogen peroxide solution at 20°C for 1 hour, and defatized by treating it in 20% aqueous acetone solution at 20°C for 1 hour.

### 5. Second inactivation step

Subsequently, the tissue specimen was immersed in a mixed solution containing 70% concentration of n-propanol and 0.2% peracetic acid in distilled water, and treated at 20°C for 4 hours and a half to perform a second inactivation step.

Finally, the tissue specimen subjected to all of the processing steps was washed with PBS solution and distilled water to prepare the multi-stage decellularized tissue supplement of Example 1.

### [Experimental Example 1]

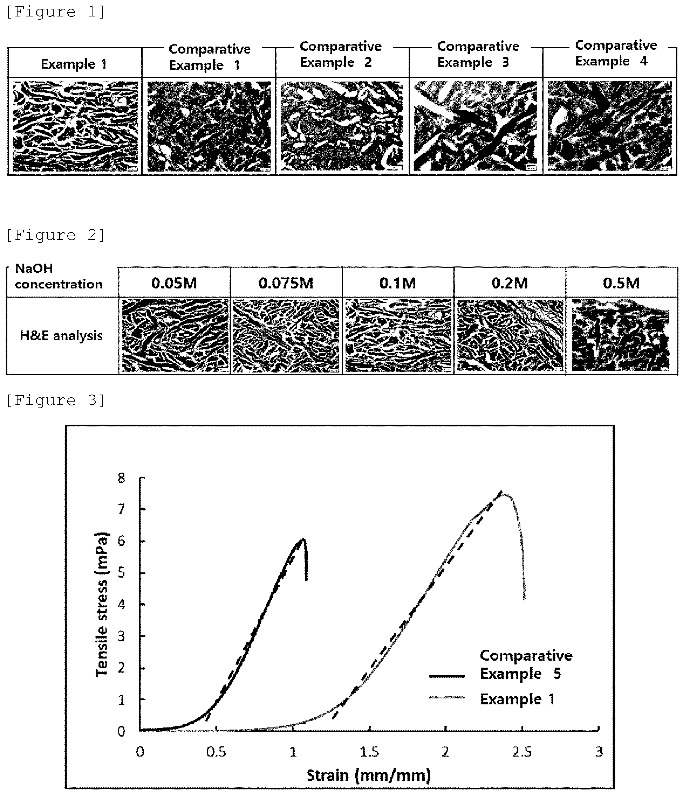

Tissue supplements were prepared using the same method as in the Preparative Example, but tissue supplements of Comparative Examples 1 to 4 were prepared by treating the tissue while changing the concentration of sodium hydroxide in the primary decellularization step without performing the secondary decellularization step, and then, H&E staining photographs of Example 1 and Comparative Examples 1 to 4 were taken and shown in Figure 1, and DNA and crude fat contents were measured and the results were listed in Table 1.

The DNA content was measured by performing the experiment according to the protocol of the assay kit using the assay kit Quant-iT^{™} PicoGreen^{™} dsDNA Assay kits and dsDNA Reagents (manufacturer: Invitrogen), and then measuring the absorbance at 480-520 mm using the Multimode plate reader Victor Nivo^{™} (manufacturer: Perkin Elmer).

The crude fat content was measured using the Sohxlet extraction method in ISO 1444 Methods of test for Meat and meat products-Part 5. Determination of free fat content.

**[Table 1]**

| | Example 1 | Comparativ e Example 1 | Comparativ e Example 2 | Comparativ e Example 3 | Comparativ e Example 4 |
|---|---|---|---|---|---|
| Secondary decellularization step | Implemented | Not implemented | | | |
| NaOH concentration (M) | 0.1 | 0.01 | 0.05 | 0.1 | 0.5 |
| H&E analysis results | No nucleus observed | Multiple nuclei observed | Multiple nuclei observed | Multiple nuclei observed | Multiple nuclei observed |
| DNA content (ng/mg) | 0.69 | 344 | 45.5 | 13.6 | 89.2 |
| Crude fat content (%) | 0.22 | 0.31 | 0.21 | 0.30 | 0.20 |

Referring to Table 1 and Figure 1, it can be confirmed that when both the primary and secondary decellularization steps are performed as in Example 1, cell nuclei are not observed and the DNA and crude fat contents are very low. On the other hand, it was confirmed that when the secondary decellularization step was not performed as in Comparative Examples 1 to 4, decellularization progressed to a certain level as the concentration of sodium hydroxide contained in the treatment solution increased, but decellularization did not progress completely overall, and therefore, a large number of cell nuclei were observed (see the parts indicated by the arrow in the photographs of Comparative Examples 1 to 4 in Figure 1), and the DNA content was also at a high level.

Therefore, from the results of this experiment, it can be confirmed that both primary and secondary decellularization processes are necessary.

### [Experimental Example 2]

Tissue supplements were prepared using the same method as in the Preparative Example, but the tissue supplements were prepared by treating the tissue while changing the concentration of sodium hydroxide in the primary decellularization step, and then, H&E staining photographs of each tissue supplement were taken and shown in Figure 2, and collagenase degradation time, tensile stress, suture strength, DNA and crude fat contents were measured and listed in Table 2.

The collagenase degradation time was determined by preparing 0.5×0.5 cm tissue supplement specimens, treating each sample with 1 ml of collagenase diluted to 100 U/ml, and measuring the time required for the tissue supplement specimens to completely degrade. Three experiments were performed for each tissue supplement, and the average value was determined as the collagenase degradation time.

The tensile stress was measured using a test method based on the ASTM D638 standard, and the specimens were cut to fit Dog-bone type 5, and the specimens were clamped in a jig with an interval of 2.54 cm and then tensioned at a speed of 10 mm/min for measurement. Five specimens were prepared for each tissue supplement to perform the test.

The suture strength was measured according to the ISO7198 standard. The specimens were prepared by cutting to a size of 2×4 cm, and the suture was inserted so that it passed vertically 2 mm from the end of the specimen, and the specimen and suture were each bitten by each jig, and then were pulled at a speed of 50 to 200 mm/min for measurement. Five specimens were prepared for each tissue supplement to measure the suture strength, and the average of the measured values was determined as the suture strength of the corresponding tissue supplement.

**[Table 2]**

| NaOH content (M) | 0.05 | 0.075 | 0.1 | 0.2 | 0.5 |
|---|---|---|---|---|---|
| H&E analysis results | Nucleus removed | Nucleus removed | Nucleus removed | Nucleus removed | Nucleus removed, tissue collapse observed |
| Collagenase degradation time | Within 720 hours | Within 480 hours | Within 120 hours | Within 96 hours | Within 4-6 hours |
| Tensile stress (Mpa) | 20-30 | 20-30 | 10-20 | 5-10 | 1 or less |
| Suture strength (N) | 78.15 | 70.51 | 23.07 | 14.98 | 3.19 |
| DNA content (ng/mg) | 17.12 | 17.37 | 0.69 | 0.51 | 0.15 |
| Crude fat content (%) | 0.94 | 0.37 | 0.22 | 0.22 | 0.3 |

Referring to the experimental results in Table 2 and Figure 2, first, it can be confirmed that as the concentration of sodium hydroxide increases in the primary decellularization step, the mechanical properties, suture strength and degradation resistance against collagenase decrease. In addition, it can be confirmed that as the concentration of sodium hydroxide increases, the DNA content decreases, and thus, the decellularization efficiency increases, and it can be seen that the DNA content in all experimental groups is 50 ng/mg or less. In general, different degradation periods and mechanical properties are required for each site into which the tissue supplement is to be transplanted, and from the experimental results above, it was confirmed that the tensile stress, suture strength and collagenase degradation time may be controlled by changing the NaOH concentration in the primary decellularization step, and therefore, it could be confirmed that the tissue supplement according to the present invention may be customized to a transplant site that requires different physical properties and degradation periods.

In addition, when the concentration of sodium hydroxide was 0.05 to 0.2 M, no tissue collapse was observed, but when 0.5 M was used, tissue collapse was observed. Therefore, from the results of this experiment, it could be confirmed that the concentration of sodium hydroxide was 0.05 M or more and less than 0.5 M in the primary decellularization step to obtain a tissue supplement with appropriate strength and degradation time and with sufficient decellularization.

### [Experimental Example 3]

A tissue supplement was prepared using the same method as in the Preparative Example, but the tissue supplement of Comparative Example 5 by using ethanol as the alcohol in the first decellularization step.

Thereafter, the tensile stress depending on the strain of the tissue supplements of Example 1 and Comparative Example 5 was measured, and the results were shown in Figure 3, and the modulus (slope) values were obtained and listed in Table 3.

**[Table 3]**

| | Comparative Example 5 | Example 1 |
|---|---|---|
| Alcohol type | Ethanol | n-Propanol |
| Tensile stress (Mpa) | 6.04 | 7.48 |
| Strain (mm/mm) | 1.004 | 1.930 |
| Modulus (slope) | 9.80 | 6.98 |

Referring to the results in Table 3 above and Figure 3, it could be confirmed that Example 1 showed higher tensile stress and strain than Comparative Example 5, and thus, the mechanical properties of Example 1 were superior. In addition, it was confirmed that in the slope indicating the degree of softness, Example 1 was gentler than Comparative Example 5, and thus, Example 1 was a softer material, i.e., a material more similar to the human body. Therefore, from the results of this experiment, it could be confirmed that it is preferable to use n-propanol as the alcohol contained in the treatment solution in the first decellularization step.

### [Experimental Example 4]

Tissue supplements were prepared using the same method as in the Preparative Example, but tissue supplements of Example 2 and Comparative Example 6 were prepared by changing the concentration of sodium hydroxide used in the primary decellularization step to 0.05 M, and the stirring speed throughout the entire process to 50 RPM and 150 RPM, respectively.

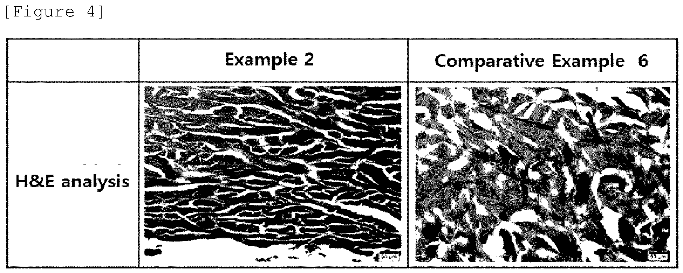

Thereafter, H&E staining photographs of the two tissue supplements were taken and shown in Figure 4, and collagenase degradation time, tensile stress, DNA content and crude fat content were measured using the same method as in Experimental Example 2, and the results are listed in Table 4.

**[Table 4]**

| | Example 2 | Comparative Example 6 |
|---|---|---|
| H&E analysis results | Nuclear removed | Nuclear removed, tissue damage |
| Collagenase degradation time | Within 240 hours | Within 24 hours |
| Tensile stress (Mpa) | 15-20 | 1-1.5 |
| DNA content (ng/mg) | 5.130 | 43.120 |
| Crude fat content (%) | 0.94 | 0.84 |

Referring to Table 4 and Figure 4, it could be confirmed that as the stirring speed increased, the damage to the tissue became more severe, and accordingly, the mechanical properties were lowered, the degradation time decreased, and the DNA removal efficiency actually decreased. Therefore, from the results of this experiment, it could be confirmed that high stirring speed caused physical damage to the tissue during the process of preparing tissue supplements, and specifically, it could be confirmed that it is preferable to perform the entire process at a stirring speed of less than 150 RPM.

The present invention is not limited to the specific embodiments and descriptions described above, and various modifications may be made by those of ordinary skill in the technical field to which the present invention pertains without departing from the gist of the present invention as claimed in the claims, and such modifications fall within the protection scope of the present invention.

### [Industrial Availability]

The present invention relates to a multi-stage decellularized tissue supplement having low DNA content and crude fat content and capable of controlling mechanical properties and degradation time by multi-stage decellularization, and a method for preparing the same. It may be customized to the human organ into which the tissue supplement is to be transplanted because the DNA content and crude fat content are low and the degradation time and physical strength may be controlled. In addition, it has the advantage of being able to process about twice as much at one time as the conventional method because the decellularization efficiency is improved through the multi-stage decellularization process, and remarkably improving productivity because the processing time is short. In addition, it is able to remove most of the DNA in a short period of time even when a lower concentration of DNA-degrading enzyme is used because the decellularization efficiency is improved. Therefore, it has industrial applicability.

## Claims

1. A multi-stage decellularized mammal-derived tissue supplement, prepared by a method comprising:
a preparation step for preparing tissue of a non-human mammal;
a pretreatment step for pretreating the tissue;
a first inactivation step for inactivating a virus included in the pretreated tissue using alcohol;
a primary decellularization step for removing cells from the virus-inactivated tissue using an aqueous basic solution;
a secondary decellularization step for removing cells by enzymatically treating the primary decellularized tissue; and
a second inactivation step for inactivating a virus included in the decellularized tissue using an acid, and
wherein the DNA content in the tissue subjected to the pretreatment step to the second inactivation step is 50 ng/mg or less,
wherein the degradation time using 100 U/ml of collagenase is at least 90 hours, and
wherein the primary decellularization step comprises a first decellularization step performed using a mixture of n-PrOH and NaOH; and a second decellularization step performed using NaOH of 0.05 M or more and less than 0.5 M.

2. The multi-stage decellularized mammal-derived tissue supplement according to claim 1, **characterized in that** the tissue is any one or more of mammal-derived skin, cartilage, ligament, tendon, myocardial membrane, pericardium, small intestine, bladder, liver, heart, lung, pancreas, spleen, kidney, stomach, bronchus, uterus, placenta and nerve.

3. The multi-stage decellularized mammal-derived tissue supplement according to claim 1, **characterized in that** the secondary decellularization step is performed using DNase of less than 0.03 wt%.

4. A method for preparing a multi-stage decellularized mammal-derived tissue supplement, comprising:
a preparation step for preparing skin tissue of a non-human mammal;
a pretreatment step for pretreating the tissue;
a first inactivation step for inactivating a virus included in the pretreated tissue using alcohol;
a primary decellularization step for removing cells from the virus-inactivated tissue using an aqueous basic solution;
a secondary decellularization step for removing cells by enzymatically treating the primary decellularized tissue; and
a second inactivation step for inactivating a virus included in the decellularized tissue using an acid, and
wherein the DNA content in the tissue subjected to the pretreatment step to the second inactivation step is 50 ng/mg or less,
wherein the degradation time using 100 U/ml of collagenase is at least 90 hours, and
wherein the primary decellularization step comprises a first decellularization step performed using a mixture of n-PrOH and NaOH; and a second decellularization step performed using NaOH of 0.05 M or more and less than 0.5 M.

5. The method for preparing a multi-stage decellularized mammal-derived tissue supplement according to claim 4, **characterized in that** a neutralization step using an acidic solution and a washing step using PBS are further performed between the primary and secondary decellularization steps.

6. The method for preparing a multi-stage decellularized mammal-derived tissue supplement according to claim 4, **characterized in that** a decolorization step and/or a defatization step is further performed between the secondary decellularization step and the second inactivation step.

7. The method for preparing a multi-stage decellularized mammal-derived tissue supplement according to claim 4, **characterized in that** the second inactivation step is performed using a mixture of an organic acid and an alcohol.

8. The method for preparing a multi-stage decellularized mammal-derived tissue supplement according to claim 4, **characterized in that** washing, processing, packaging and sterilization steps are performed after the second inactivation step.

9. The method for preparing a multi-stage decellularized mammal-derived tissue supplement according to claim 4, **characterized in that** the pretreatment step to the second inactivation step are performed in a stirring environment of less than 150 rpm.
